# FASCICULE DE BREVET EUROPEEN

(11) **EP 0 827 375 B1**
(45) Date de publication et mention de la délivrance du brevet: **09.02.2005**
(21) Numéro de dépôt: 97915522.3
(22) Date de dépôt: 20.03.1997
(51) Int. Cl.: A01K 67/033

(54) **PROCEDE D'ELEVAGE ET DE CONDITIONNEMENT D'AUXILIAIRES POUR LA LUTTE BIOLOGIQUE CONTRE LES RAVAGEURS DES PLANTES**
VERFAHREN ZUR ZUCHT UND VERPACKUNG VON NUTZORGANISMEN FUR DIE BIOLOGISCHEBEKAMPFUNG VON PFLANZENSCHADLINGEN
METHOD FOR BREEDING AND PACKAGING AUXILIARY ORGANISMS FOR BIOCONTROLLING PLANT PESTS

(30) Priorité: 21.03.1996 FR 9603513
(43) Date de publication de la demande: 11.03.1998
(73) Titulaire: Biotop, 75782 Paris Cédex 16 (FR); INSTITUT NATIONAL DE LA RECHERCHE AGRONOMIQUE (INRA), 75338 Paris Cédex 07 (FR)
(72) Inventeur: FRANDON, Jacques, F-06600 Antibes (FR); FRENOY-EL BAZE, Corinne, Villa des Glycines, F-06600 Antibes (FR); MILLOT, Pierre, F-06600 Antibes (FR); BRUN, Jacques, F-06650 Opio (FR)
(74) Mandataire: Breese, Pierre
(86) Numéro de dépôt international: PCT/FR1997/000490
(87) Numéro de publication internationale: WO 1997/034468

(56) Documents cités:
- DE-A- 4 108 031
- DE-U- 9 311 037
- GB-A- 2 000 007
- GB-A- 2 168 680

## Description

La présente invention concerne un procédé d'élevage et de conditionnement d'auxiliaires pour la lutte biologique contre les ravageurs des plantes.

De façon générale, le développement de la lutte biologique contre les ravageurs des cultures a été rendu possible grâce à la mise au point de méthodes de production industrielle d'auxiliaires insectes ou acariens. Ces productions d'organismes utiles se font le plus souvent à partir de proies ou d'hôtes naturels, de proies ou d'hôtes de substitution ou, plus rarement, de proies ou d'hôtes artificiels. La rentabilité de telles productions n'est pas toujours évidente car, d'une part, elles demandent des investissements lourds nécessités par les processus biologiques qu'il faut parfaitement maîtriser et, d'autre part, l'automatisation des productions est difficile et parfois impossible et, enfin, la productivité des élevages est souvent faible.

En effet, dans les élevages industriels, il y a modification du comportement des arthropodes lorsqu'ils se retrouvent trop nombreux dans un espace donné. Il apparaît alors des phénomènes de masse, des compétitions intraspécifiques, qui ont pour conséquence des pertes quantitatives et/ou qualitatives dans les élevages, par exemple le superparasitisme chez les parasites et surtout le cannibalisme chez les prédateurs. Ces comportements, qui existent naturellement chez la plupart des espèces dans leur milieu naturel mais sont exacerbés par les conditions d'élevage, entraînent une diminution du nombre d'individus produits par unité de surface ou de volume et donc aussi une diminution de la productivité de l'élevage. Ces pertes, qui sont variables d'une espèce à l'autre, sont d'autant plus marquées que le nombre d'individus par unité de volume est important.

Dans le cas des arthropodes prédateurs, ces phénomènes dépresseurs interviennent aussi et fortement au niveau des conditionnements destinés aux utilisateurs. En effet, afin de limiter les coûts de manutention, d'emballage, de transport et d'utilisation, les organismes utiles sont souvent conditionnés en grand nombre dans de petits volumes et la plupart du temps sans nourriture à leur disposition ou avec une nourriture difficilement accessible. Il s'ensuit des pertes d'individus par cannibalisme et aussi une baisse de la qualité, qui peuvent être parfois très importantes si le stockage ou le transport est trop long. Le conditionnement des arthropodes dans la demande de brevet GB 2168680 se fait dans une enveloppe à l'intérieur de laquelle est deposé un matériel servant de nourriture aux arthropodes.

Ces difficultés d'élevage et d'utilisation sont bien illustrées dans le cas particulier de la coccinelle Harmonia axyridis. Celle-ci est un insecte prédateur qui se nourrit de pucerons. Elle est très intéressante de part sa grande polyphagie, c'est-à-dire qu'elle consomme plusieurs espèces de pucerons, et sa très bonne capacité prédatrice aussi bien au stade larvaire qu'au stade adulte. Cependant, ces qualités de prédatisme se trouvent être un inconvénient dans l'élevage de masse à cause du risque important de cannibalisme.

Cette coccinelle est habituellement multipliée sur une nourriture de substitution telle que les oeufs de la teigne de la farine, Ephestia kuehniella, que l'on sait déjà produire de façon industrielle pour l'élevage d'entomophages. Cette proie de substitution est de manutention facile du fait que les oeufs sont tous individualisés et se présentent sous forme d'une "poudre". En outre, les insectes élevés avec cette proie de substitution offrent des potentialités biologiques comparables, voire supérieures, à celles obtenues avec les proies naturelles.

La coccinelle Harmonia axyridis est commercialisée au stade larvaire. La production des larves se fait directement dans les boîtes servant à leur conditionnement pour la vente : ces boîtes sont en plastique transparent et de dimensions variables, fermées par des couvercles, et contenant la nourriture, par exemple des oeufs d'Esphestia kuehniella, qui est nécessaire au développement des larves jusqu'au troisième stade larvaire dénommé "L3".

Jusqu'ici, la fabrication d'une boite était réalisée comme suit :
- le fond de la boîte est préalablement encollé, habituellement avec une colle acrylique étalée au pinceau,
- la dose de nourriture est versée sur le fond de la boîte et s'y colle,
- on dépose alors sur la nourriture des pontes de coccinelles,
- on referme le couvercle de la boîte,
- la boîte est disposée dans une salle climatisée pour le développement des insectes.

En fonction de la taille des boites actuelles, on peut produire de 20 à 200 larves environ par boîte.

Cette méthode permet d'obtenir des rendements intéressants, mais elle présente plusieurs inconvénients :
- les boîtes étant fermées, il peut y avoir un excès d'humidité à l'intérieur dû à la présence de la nourriture et au métabolisme propre des larves. Une sorte de fermentation peut alors s'opérer dans la boîte avec une dégradation de la nourriture qui entraîne une mortalité des larves par manque de nourriture ou cannibalisme et une baisse de leur qualité,
- la surface sur laquelle on colle la nourriture, c'est-à-dire le fond de la boite, étant limitée, la quantité de larves pouvant normalement se développer jusqu'au stade larvaire L3 est elle aussi limitée. Toute tentative d'augmenter le nombre de larves produites dans le même volume entraîne une augmentation du cannibalisme et donc une diminution de la productivité de l'élevage.

Pour pouvoir produire plus de larves par unité de conditionnement, il faut alors passer à des dimensions supérieures pour les boites, ce qui entraîne un surcoût et des difficultés supplémentaires pour les lâchers.

A cet égard, les larves de la coccinelle Harmonia axyridis sont utilisées actuellement par les jardiniers amateurs pour lutter contre les pucerons des rosiers ou d'autres pucerons sur les cultures ornementales ou maraîchères. Il est également envisagé de les utiliser dans le milieu profesionnel, en protection intégrée sous serre ou en arboriculture.

La difficulté des lâchers de larves réside dans le fait qu'elles sont produites dans des boites de conditionnement importantes (de 20 à 200 larves) alors que quelques individus seulement sont nécessaires sur chaque végétal, par exemple 1 à 2 larves par tige de rosier.

Pour les particuliers, l'obstacle a été en partie contourné en fournissant avec les boîtes de larves un petit pinceau qui permet de prendre les larves une à une et de les déposer sur les plantes.

Cependant, une telle méthode ne peut pas être utilisée dans le milieu professionnel où il s'agit de lâcher les larves sur de grandes surfaces, de façon homogène, et dans un laps de temps minimal.

Les boîtes de production de larves actuellement utilisées ne sont donc pas du tout compatibles avec les besoins du milieu professionnel.

Les recherches qui ont conduit à l'invention ont montré de façon surprenante que l'on pouvait obtenir une diminution des pertes de l'élevage de masse dans les conditionnements, qu'ils s'agisse de boîtes ou de sachets, tout en obtenant, d'une part, une amélioration de la qualité des auxiliaires par de meilleures conditions d'élevage et, d'autre part, une plus grande facilité d'utilisation.

A cet égard, il y a lieu de noter que le terme "boîte" utilisé dans le présent mémoire désigne aussi bien un sachet que tout autre type de conditionnement. De la même manière le terme "grains de maïs éclatés" désigne aussi bien des produits tels que cartons ou papiers broyés et conformés ou non en boulettes, grains ou autres formes de polystyrène expansé du genre destiné à l'emballage, cosses de sarrasin, vermiculite, perlite, son, copeaux de bois ou tout autre support de préférence léger, offrant la surface maximale pour un certain volume et pouvant être enduit de nourriture comme indiqué dans le présent mémoire.

Conformément à l'invention, le procédé d'élevage et de conditionnement d'auxiliaires pour la lutte biologique contre les ravageurs des plantes est du type consistant à sélectionner une ou des boîtes ou sachets de conditionnement ayant les dimensions appropriées pour élever approximativement le nombre recherché d'individus d'un auxiliaire déterminé, à introduire dans la ou les boîtes la dose de nourriture spécifique nécessaire à l'élevage de cet auxiliaire, à déposer sur la nourriture un nombre approprié de pontes ou d'inoculum dudit auxiliaire, à fermer le couvercle de la ou des boites, à disposer la ou les boîtes fermées dans un local éclairé et climatisé pour le développement de l'auxiliaire pendant plusieurs jours jusqu'à ce qu'il atteigne le stade désiré, puis à transporter sur le lieu de lâcher la ou les boîtes contenant l'auxiliaire développé jusqu'audit stade désiré et à ouvrir la ou les boîtes pour procéder au lâcher.

Selon une caractéristique essentielle de l'invention, on sélectionne des supports tels que des grains de maïs éclatés, on en mesure la quantité nécessaire pour remplir la ou les boites, on enduit la surface de ces grains de maïs éclatés d'un produit d'encollage, on réunit ensemble la dose de nourriture spécifique et la quantité nécessaire de grains de mais éclatés préencollés, on colle cette dose de nourriture sur les grains de mais éclatés préencollés en la répartissant par secouage, on dépose le nombre approprié de pontes ou d'inoculum de l'auxiliaire sur les grains de maïs éclatés enduits de nourriture, on ferme le couvercle de la ou des boites, que l'on transfère dans le local éclairé et climatisé, et on laisse les auxiliaires issus des pontes ou de l'inoculum se multiplier et se développer sur les grains de mais éclatés enduits de nourriture jusqu'à ce qu'elles atteignent le stade désiré pour le lâcher, grâce à quoi les grains de maïs éclatés constituent à la fois le support de la nourriture, un excipient permettant d'améliorer les conditions d'élevage, en particulier au niveau de l'humidité, et fournissant une très importante surface disponible pour le développement de chaque auxiliaire, et un support de lâcher sur lequel en moyenne quelques individus se maintiennent encore après l'ouverture de la ou des boîtes en vue dudit lâcher, constituant ainsi un micro conditionnement tout à fait adapté à une bonne répartition des auxiliaires sur les plantes.

Selon d'autres caractéristiques préférées de l'invention :
- le produit d'encollage est une huile végétale telle que l'huile de tournesol, d'arachide ou d'olive, par exemple, et l'on enduit la surface des grains de maïs éclatés, de préférence par brassage dans un récipient rotatif à axe excentré,
- ou bien le produit d'encollage est une colle, par exemple du type acrylique, et l'on enduit la surface des grains de maïs éclatés, de préférence par pulvérisation,
- ou encore le produit d'encollage est du sucre caramélisé ou du miel liquide et l'on enduit la surface des grains de mais éclatés par brassage à air pulsé dans un récipient fixe.

Les grains de maïs éclatés enduits de sucre caramélisé peuvent évidemment être des grains de mais éclatés "alimentaires" achetés directement dans le commerce, mais ils présentent alors des formes généralement beaucoup plus irrégulières que celles des grains de maïs éclatés destinés au calage de produits dans les emballages et provenant de l'agriculture. De ce fait, lorsque le produit d'encollage est du sucre caramélisé, on préfère employer comme support des grains de maïs éclatés "emballage", dont les dimensions plus régulières permettent une distribution plus homogène du nombre de larves par grain de maïs éclaté. Par ailleurs, les grains de maïs éclatés du type emballage sont habituellement utilisés comme support lorsque le produit d'encollage est une huile végétale, ou une colle, ou encore du miel liquide tel que le miel d'acacias.

Selon encore d'autres caractéristiques de l'invention :
- dans le cas de petites productions, on détermine la quantité de nourriture spécifique nécessaire en fonction du nombre d'auxiliaires à développer dans chaque boîte considérée, on dépose cette quantité de nourriture sur le fond de chaque boîte considérée, on ajoute dans chaque boîte la quantité préalablement mesurée de grains de maïs éclatés préencollés, on ferme chaque boîte, on la secoue pour coller la nourriture sur les grains de maïs éclatés préencollés, et on la rouvre pour y déposer le nombre, approprié de pontes de l'auxiliaire.
- ou bien, dans le cas de grandes productions, on calcule la quantité moyenne de nourriture spécifique nécessaire que l'on veut coller par unité de volume des grains de mais éclatés préencollés, on en déduit une quantité totale de nourriture spécifique que l'on dépose dans un grand récipient intermédiaire, on ajoute dans ce récipient la quantité totale calculée de grains de maïs éclatés préencollés, on effectue un secouage dudit récipient pour coller la nourriture sur les grains de mais éclatés préencollés, on transfère du récipient dans chaque boite d'élevage la quantité de nourriture voulue déjà collée sur les grains de mais éclatés, on laisse chaque boite ouverte et l'on y dépose le nombre approprié de pontes ou d'inoculum de l'auxiliaire.

De préférence, on choisit comme nourriture spécifique les oeufs de la teigne de la farine, Esphestia kuehniella, et l'on choisit comme auxiliaire, soit des insectes entomophages tels que, par exemple, les chrysopes, les punaises prédatrices, les trichogrammes et, de préférence, la coccinelle Harmonia axyridis, soit un acarien.

En se référant à nouveau à la coccinelle Harmonia axyridis déjà prise à titre d'exemple ci-dessus et nourrie avec des oeufs de la teigne de la farine, Ephestia kuehniella, on constate que les grains de mais éclatés mis en oeuvre selon l'invention permettent d'obtenir des améliorations importantes dans plusieurs domaines indiqués ci-après.

### Les grains de maïs éclatés enduits de nourriture améliorent les conditions d'ambiances dans les boîtes de production de larves.

En effet, dans les boîtes d'élevage de larves de coccinelles, qui sont fermées, la présence des oeufs d'Ephestia kuehniella et le métabolisme des larves entraînent rapidement une élévation importante de l'hygrométrie ambiante. Ceci peut provoquer des développements de moisissures et aussi déclencher une sorte de fermentation dans les boîtes, qui a pour conséquence une dégradation plus rapide des oeufs d'Ephestia kuehniella qui deviennent marrons et ne sont plus acceptés par les larves. En plus de ce changement de couleur des oeufs, on remarque également une odeur désagréable lorsqu'on ouvre les boîtes. A partir de ce moment, les larves de coccinelles n'ont plus assez de nourriture disponible et l'on observe une croissance ralentie, de nombreuses larves deviennent rachitiques, d'autres meurent et il y a aussi augmentation du cannibalisme. Finalement, il y a diminution de la productivité de l'élevage et une baisse importante de la qualité des insectes produits et ceci d'autant plus que le nombre de larves par boîte est plus élevé.

Lorsqu'on ajoute des grains de maïs éclatés enduits de nourriture dans les boîtes, il n'y a pas d'augmentation de l'hygrométrie car ils ont la capacité d'absorber le surplus d'hygrométrie. Ils jouent donc le rôle d'absorbeurs et, par conséquent, ils tamponnent les conditions abiotiques dans les boites. Les oeufs d'Ephestia kuehniella se conservent bien et restent parfaitement disponibles pour les larves, qui ont un développement normal et homogène, avec un minimum de cannibalisme. Ceci permet aussi une meilleure conservation des larves car elles ont une nourriture de qualité sur une longue période.

### Les grains de maïs éclatés enduits de nourriture diminuent le cannibalisme dans les boîtes.

Cette diminution vient tout d'abord du fait que la nourriture reste disponible en quantité et en qualité pendant toute la durée de développement des larves, pour les raisons exposées ci-dessus.

Elle vient aussi du fait que les grains de maïs éclatés augmentent considérablement la surface disponible pour les larves, qui est multipliée par cinq dans une boîte standard et qui augmente encore en fonction des dimensions de la boîte. En outre, cette surface présente de nombreuses discontinuités. L'augmentation de la surface et la présence de discontinuités permettent aux larves de se cacher et d'éviter les rencontres avec leur congénères.

De plus, les oeufs d'Ephestia kuehniella étant collés sur les grains de maïs éclatés, ils sont donc disponibles dans tout le volume de la boite, et non plus uniquement sur le fond. Ils se trouvent à proximité immédiate des larves, quelle que soit leur position, ce qui, là encore, évite des déplacements pour la recherche de nourriture et les pertes d'énergie et de temps de développement ainsi que la rencontre avec les autres larves. Il en résulte une forte diminution du cannibalisme dans les boites et donc une meilleure productivité.

En outre, les grains de maïs éclatés enduits de nourriture permettent d'augmenter le nombre d'individus produits par unité de volume. Les larves peuvent se développer dans des conditions optimales et de façon très homogène, ce qui donne un produit de meilleure qualité. Le nombre de boite mises au rebut (quantité de larves insuffisante, par exemple) diminue, ce qui améliore encore la productivité de l'élevage.

### Les grains de maïs éclatés enduits de nourriture constituent un microconditionnement pratique.

En effet, la majorité des larves reste sur les grains de mais éclatés enduits de nourriture lorsqu'elles se développent dans les boîtes et elles sont réparties de façon homogène dans l'ensemble du volume. Chaque grain de mais éclaté enduit de nourriture constitue donc un microsupport sur lequel on peut trouver de une à plusieurs larves en fonction de la densité des individus dans la boite et de la taille des grains de maïs.

Ces supports peuvent être utilisés pour déposer des larves de coccinelles en très petit nombre directement sur les végétaux et de manière rapide, ce qu'il est impossible de réaliser avec les boites classiques sans grains de mais éclatés, qui contiennent de 20 à 200 larves, à moins d'utiliser un pinceau, ce qui est particulièrement long.

L'épandage des larves de coccinelles sur de grandes surfaces à raison de quelques individus par m² devient tout à fait possible en répartissant simplement les grains de maïs éclatés sur la surface des plantes. Cette opération peut se faire d'autant plus aisément que les larves ne quittent pas les boîtes une fois ouvertes mais restent en très grande majorité sur les grains de mais éclatés enduits de nourriture pendant la durée de l'épandage.

En conclusion, les grains de mais éclatés enduits de nourriture améliorent considérablement la production, le conditionnement et l'utilisation des larves de coccinelles. Mais cette technique permet aussi d'améliorer les productions d'autres auxiliaires utilisés en lutte biologique (chrysopes, punaises prédatrices, trichrogrammes, acariens, ...), qui sont soumises aux mêmes difficultés.

En outre, les grains de maïs éclatés enduits de nourriture constituent un produit très léger, ce qui est un grand avantage par rapport aux risques de blessures des auxiliaires pendant les manutentions et aussi lors de l'épandage sur les cultures.

A cet égard, il est préférable que les boites d'élevage et de conditionnement soient remplies à ras bords de grains de mais éclatés, de telle sorte qu'après la fermeture du couvercle lesdits grains de mais ne risquent pas de se déplacer et de blesser les larves lors du transport des boites.

Par ailleurs, ce produit est d'origine naturelle et parfaitement biodégradable. Il s'agit en fait d'une utilisation supplémentaire d'un produit d'origine agricole pour un usage agricole. Sa production et son utilisation n'entraînent absolument aucune nuisance pour le personnel ou pour l'environnement.

Les résultats de différents essais comparatifs sont résumés dans les tableaux ci-après, dans lesquels :
- J représente le jour du premier comptage des larves, considéré comme le jour de début des essais,
- répétitions/modalité signifie que l'on a effectué le même essai avec un nombre de boites correspondant aux "répétitions", et ce pour chaque modalité avec ou sans mais,
- L1, L3, L4 représentent respectivement les premier, troisième et quatrième stades larvaires, c'est-à-dire différents stades du développement des larves,
- sans mais signifie que la nourriture est collée directement sur le fond de la boîte selon la technique antérieure,
- avec mais signifie que l'on emploie des grains de mais éclatés enduits de nourriture,
- e. k. représente Ephestia kuehniella.

### A. INTERET DES GRAINS DE MAIS COMME SUPPORT DE NOURRITURE

### 1. Elevage dans petites barquettes

Nourriture suffisante
6 pontes/barquette
5 répétitions/modalité

| Modalités | J | J + 3 | | J + 7 | | Pertes entre J+3/J+7 |
|---|---|---|---|---|---|---|
| | Nb larves L1 | Nb larves | % de L3 | Nb larves | % de L4 | |
| Sans maïs | 98,6 | 96,6 | 58,5 % | 43,6 | 14,7 % | - 54 % |
| Avec maïs | 101,3 | 89,4 | 91,3 % | 60 | 53,7 % | - 33 % |

### 2. Elevage dans petites boîtes plates

Nourriture suffisante
3 pontes par boîte
5 répétitions/modalité

| Modalités | J + 6 | | J + 9 | | Parte entre L3 et L4 | Aspect des boîtes |
|---|---|---|---|---|---|---|
| | Nb larves | % de L3 | Nb larves | % de L4 | | |
| Sans maïs | 48,2 | 54,7 % | 28 | 18,6 % | - 42 % | Mauvaises odeurs dans les boîtes. Hétérogénéïté des stades |
| Avec maïs | 44,4 | 84,6 % | 33,2 | 38,5 % | - 25 % | Belles larves homogènes. Pas de mauvaises odeurs |

### 3. Elevage dans petites boîtes hautes

Nourriture suffisante
6 pontes par boîte
4 répétitions/modalité

| Modalités | J + 4 | | Aspect des boîtes |
|---|---|---|---|
| | Nbre de larves | % de L3 | |
| Sans maïs | 106,5 | 30 % | Mauvaises odeurs, oeufs e.k marrons, larves petites et rabougries |
| Avec maïs | 119,2 | 45 % | Pas de mauvaises odeurs, oeufs e.k. bien conservés, larves belles |

### 4. Elevage dans grandes boites

Nourriture suffisante
15 pontes/boîte
5 répétitions/modalité

| Modalités | Nb moyen de larves/barquette | Poids moyen des L3 (mg/L) | % de L3 |
|---|---|---|---|
| Sans maïs | 179,8 | 4,5 | 61,6 % |
| Avec maïs | 216,4 | 7,52 | 85,3 % |

### COMMENTAIRES

Avec la présence de maïs, la population de larves est plus homogène, le développement est plus rapide et il y a moins de perte par cannibalisme car il y a meilleure utilisation de la nourriture : la nourriture se conserve plus longtemps.

Les pesées de larves de stade L3 ont montré que le poids des larves est plus important quand les larves sont élevées dans des boîtes avec grains de maïs éclatés, en particulier dans le cas où la densité de larve est grande (jusqu'à plus de 60 % d'augmentation).

### B. INTERET DES GRAINS DE MAIS COMME MICRO CONDITIONNEMENT (SUPPORT DE LACHER)

### 1. Intérêt du collage de la nourriture sur les grains de maïs éclatés

### Elevage dans grandes barquettes, nouriture suffisante (oeufs d'e.k.)

| Nbre de pontes par boîtes | Modalités | % de larves sur grains de maïs |
|---|---|---|
| 6 pontes | Grains avec oeufs | 93,6 % |
| | Grains sans oeufs | 60 % |
| 9 pontes | Grains avec oeufs | 95,1 % |
| | Grains sans oeufs | 56,9 % |
| 12 pontes | Grains avec oeufs | 89 % |
| | Grains sans oeufs | 64,4 % |

### 2. Etude du nombre de larves par grain de maïs

### Elevage dans grande boîte avec des oeufs d'e.k. en quantité suffisante collés sur grain de maïs

Comptage au stade L3 :
388 larves dont 310 sur maïs (soit 80 % du total)
83 % sont au stade L3 (lot très homogène)
85 % des grains sont porteurs de larves, la grande majorité (73 %) ayant entre 1 et 4 larves

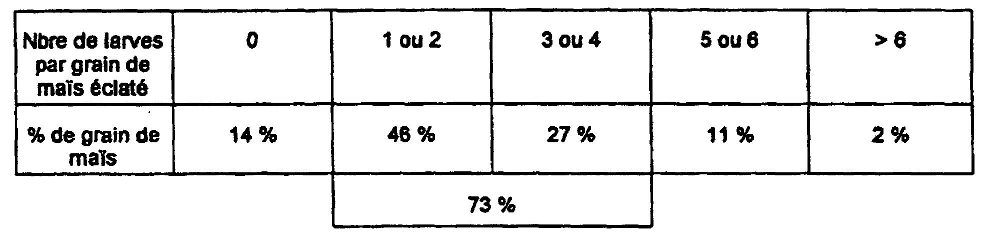

### COMMENTAIRES

Quelle que soit la densité des larves par boîte, le collage de la nourriture sur les grains de maïs permet d'avoir plus de grains de maïs avec au moins une larve. On obtient ainsi un micro conditionnement homogène très pratique pour la répartition des auxiliaires sur les plantes.

Tous les résultats des essais ci-dessus confirment bien que l'emploi des grains de maïs éclatés enduits de nourriture améliore considérablement la production des larves, tant du point de vue quantité que du point de vue qualité.

Lors du lâcher, quelques larves ne se trouvent pas sur les grains de maïs et restent sur la surface intérieure des boîtes. Il est évident que l'épandage de ces larves restantes ne peut être réalisé que par secouage in situ.

Il est bien entendu que la présente invention n'a été décrite qu'à titre explicatif dans le cas d'Harmonia axyridis mais nullement limitatif et qu'on pourra y apporter toute modification utile, notamment dans le domaine des équivalences techniques, sans sortir de son cadre.

## Revendications

1. Procédé d'élevage et de conditionnement d'auxiliaires de culture choisis parmi les insectes entomophages ou les acariens pour la lutte biologique contre les ravageurs des plantes, **caractérisé par le fait que** ledit procédé présente les étapes suivantes :
- on sélectionne des grains de mais éclatés,
- on en remplit une boîte,
- on enduit la surface de ces grains de mais éclatés d'un produit d'encollage,
- on réunit ensemble de la nourriture de substitution et les grains de mais éclatés préencollés,
- on colle cette nourriture sur les grains de mais éclatés préencollés en la répartissant par secouage,
- on dépose des pontes ou un inoculum de l'auxiliaire sur les grains de mais éclatés enduits de nourriture,
- on ferme le couvercle de la ou des boîtes,
- on transfère dans un local éclairé et climatisé ladite boîte,
- on laisse les auxiliaires issus des pontes ou de l'inoculum se multiplier et se développer sur les grains de maïs éclatés enduits de nourriture jusqu'à ce qu'elles atteignent le stade désiré pour les lâcher.

2. Procédé suivant la revendication 1, **caractérisé par le fait que** le produit d'encollage est une huile végétale et qu'on enduit la surface des grains de mais éclatés par brassage dans un récipient rotatif à axe excentré.

3. Procédé suivant la revendication 1, **caractérisé par le fait que** le produit d'encollage est une colle du type acrylique et qu'on enduit la surface des grains de maïs éclatés par pulvérisation.

4. Procédé suivant la revendication 1, **caractérisé par le fait que** la produit d' encollage est du sucre caramélisé ou du miel liquide et qu'on enduit la surface des grains de mais éclatés par brassage à air pulsé dans un récipient fixe.

5. Procédé suivant l'une quelconque des revendications 1 à 4, **caractérisée en ce que** l'on dépose la nourriture au fond de la boîte, avant d'ajouter les grains de mais éclatés préencollés et de secouer pour coller la nourriture sur les grains de mais éclatés.

6. Procédé suivant l'une quelconque des revendications 1 à 4, **caractérisé en ce que** l'on on réunit ensemble la nourriture et les grains de maïs éclatés préencollés dans un grand récipient intermédiaire, avant de transférer du récipient dans la boîte d'élevage les grains de maïs éclatés sur lesquels la nourriture est collée.

7. Procédé suivant l'une quelconque des revendications 1 à 6, **caractérisé par le fait qu'**on choisit comme nourriture les oeufs de la teigne de la farine, Ephestia Kuehniella.

8. Procédé suivant l'une quelconque des revendications 1 à 7, **caractérisé par le fait qu'**on choisit comme auxiliaire des insectes entomophages tels que par exemple, les chrysopes, les punaises prédatrices, les trichogrammes.

9. Procédé suivant la revendication 8, **caractérisé par le fait qu'**on choisit comme auxiliaire insecte de préférence la coccinelle Harmonia axyridis.

10. Procédé suivant l'une quelconque des revendications 1 à 7, **caractérisé par le fait qu'**on choisit comme auxiliaire un acarien.

11. Utilisation de grains de maïs éclatés pour l'élevage et le conditionnement d'auxiliaires pour la lutte biologique contre les ravageurs des plantes selon le procédé de la revendication 1.

## Claims

1. Method for breeding and packaging of crop auxiliaries selected from entomophagous insects or mites for biological control over plant pests, **characterised by** the fact that the said method comprises the following steps:
- popcorn grains are selected;
- a box is filled with them;
- the surface of these popcorn grains is coated with a binding product,
- the substitution food and the popcorn grains coated with a prebinding product are brought together;
- this food is binded onto the popcorn grains coated with a prebinding product, and is distributed by shaking;
- eggs or an inoculum of the auxiliary are deposited on the popcorn grains coated with food;
- the box lid(s) is (are) closed;
- the said box is transferred into an illuminated and air-conditioned room;
- the auxiliaries derived from the eggs or the inoculum are left to multiply and develop on the popcorn grains coated with food until they reach the required stage to release them.

2. Method according to claim 1, **characterised by** the fact that the binding product is a vegetable oil and that the surface of the popcorn grains is coated by mixing in a rotary receptacle with eccentric axis.

3. Method according to claim 1, **characterised by** the fact that the binding product is an acrylic type glue and that the surface of the popcorn grains is coated by spraying.

4. Method according to claim 1, **characterised by** the fact that the binding product is caramelised sugar or liquid honey and that the surface of the popcorn grains is coated by mixing with blown air in a fixed receptacle.

5. Method according to any one of claims 1 to 4, **characterised in that** food is deposited at the bottom of the box, before adding the popcorn grains coated with a prebinding product and shaking to bind the food onto the popcorn grains.

6. Method according to any one of claims 1 to 4, **characterised in that** all food and popcorn grains coated with the prebinding product are brought together in a large intermediate receptacle, before the popcorn grains on which the food is binded are transferred from the receptacle into the breeding box.

7. Method according to any one of claims 1 to 6, **characterised by** the fact that the selected food consists of eggs laid by the Mediterranean flour moth, Ephestia Kuehniella.

8. Method according to any one of claims 1 to 7, **characterised by** the fact that the auxiliaries are chosen from entomophagous insects for example such as the green lacewings, spined soldier bugs, trichogramma.

9. Method according to claim 8, **characterised by** the fact that the chosen insect auxiliary is preferably the ladybird Harmonia axyridis.

10. Method according to any one of claims 1 to 7, **characterised by** the fact that a mite is selected as the auxiliary.

11. Use of popcorn grains for breeding and conditioning of auxiliaries for biological control over plant pests according to the method described in claim 1.

## Patentansprüche

1. Verfahren zur Aufzucht und Konditionierung von Kulturhelfern, ausgewählt unter den entomophagen Insekten oder den Milben zur biologischen Bekämpfung von Pflanzenschädlingen, **dadurch gekennzeichnet, dass** das besagte Verfahren die folgenden Schritte aufweist:
- man wählt aufgebrochene Maiskörner aus,
- man füllt damit einen Behälter,
- man beschichtet die Oberfläche dieser aufgebrochenen Maiskörner mit einem Leimungsmittel,
- man bringt Substitutionsnahrung und die vorgeleimten aufgebrochenen Maiskörner zusammen,
- man klebt diese Nahrung auf die vorgeleimten aufgebrochenen Maiskörner, indem man sie durch Rütteln verteilt,
- man legt Gelege oder ein Inokulum des Helfers auf den mit Nahrung beschichteten aufgebrochenen Maiskörnern ab,
- man schließt den Deckel des Behälters oder der Behälter,
- man verbringt den besagten Behälter in einen hellen und klimatisierten Raum,
- man lässt sich die Helfer, die aus den Gelegen oder dem Inokulum hervorgegangen sind, vermehren und auf den mit Nahrung beschichteten aufgebrochenen Maiskörnern entwickeln, bis sie das gewünschte Stadium erreicht haben, um sie freizulassen.

2. Verfahren nach Anspruch 1, **dadurch gekennzeichnet, dass** das Leimungsmittel ein Pflanzenöl ist und dass man die Oberfläche der aufgebrochenen Maiskörner durch Durchmischung in einem Drehgefäß mit außermittiger Achse beschichtet.

3. Verfahren nach Anspruch 1, **dadurch gekennzeichnet, dass** das Leimungsmittel ein Acrylleim ist und dass man die Oberfläche der aufgebrochenen Maiskörner durch Zerstäuben beschichtet.

4. Verfahren nach Anspruch 1, **dadurch gekennzeichnet, dass** das Leimungsmittel karamellisierter Zucker oder flüssiger Honig ist und dass man die Oberfläche der aufgebrochenen Maiskörner durch Durchmischung mit Umwälzluft in einem feststehenden Gefäß beschichtet.

5. Verfahren nach einem der Ansprüche 1 bis 4, **dadurch gekennzeichnet, dass** man die Nahrung auf dem Boden des Behälters ablegt, bevor man die vorgeleimten aufgebrochenen Maiskörner hinzufügt und schüttelt, um die Nahrung auf die aufgebrochenen Maiskörner zu kleben.

6. Verfahren nach einem der Ansprüche 1 bis 4, **dadurch gekennzeichnet, dass** man die Nahrung und die vorgeleimten aufgebrochenen Maiskörner in einem großen Zwischengefäß zusammenbringt, bevor man die aufgebrochenen Maiskörner, auf denen die Nahrung klebt, vom Gefäß in den Aufzuchtbehälter verbringt.

7. Verfahren nach einem der Ansprüche 1 bis 6, **dadurch gekennzeichnet, dass** man als Nahrung die Eier der Mehlmotte Ephestia kuehniella auswählt.

8. Verfahren nach einem der Ansprüche 1 bis 7, **dadurch gekennzeichnet, dass** man als Helfer entomophage Insekten wie zum Beispiel Florfliegen, Raubwanzen, Schlupfwespen wählt.

9. Verfahren nach Anspruch 8, **dadurch gekennzeichnet, dass** man als Hilfsinsekt vorzugsweise den Marienkäfer Harmoia axyridis wählt.

10. Verfahren nach einem der Ansprüche 1 bis 7, **dadurch gekennzeichnet, dass** man als Helfer eine Milbe wählt.

11. Verwendung von aufgebrochenen Maiskörnern zur Aufzucht und Konditionierung von Helfern zur biologischen Bekämpfung von Pflanzenschädlingen gemäß dem Verfahren von Anspruch 1.
